(19)

Europäisches Patentamt
European Patent Office
Office européen des brevets

(11) **EP 3 981 319 A1**

(12) **EUROPEAN PATENT APPLICATION**
published in accordance with Art. 153(4) EPC

(43) Date of publication:
**13.04.2022 Bulletin 2022/15**

(21) Application number: **20817788.1**

(22) Date of filing: **04.06.2020**

(51) International Patent Classification (IPC):
***A61B 1/04*** (2006.01)

(86) International application number:
**PCT/CN2020/094410**

(87) International publication number:
**WO 2020/244582 (10.12.2020 Gazette 2020/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.06.2019 CN 201910482794**

(71) Applicant: **Beijing Institute Of Technology Beijing 100081 (CN)**

(72) Inventors:
• **HUANG, Qiang**
  **Beijing 100081 (CN)**
• **LI, Jing**
  **Beijing 100081 (CN)**
• **ZHOU, Jiyang**
  **Beijing 100081 (CN)**
• **ZHAO, Shilei**
  **Beijing 100081 (CN)**
• **DARIO, Paolo**
  **Beijing 100081 (CN)**
• **CIUTI, Gastone**
  **Beijing 100081 (CN)**
• **ZHANG, Wenhui**
  **Beijing 100081 (CN)**

(74) Representative: **Vidon Brevets & Stratégie**
  **16B, rue de Jouanet**
  **BP 90333**
  **35703 Rennes Cedex 7 (FR)**

(54) **ACTIVE MAGNETICALLY CONTROLLED CAPSULE ROBOT DETECTION SYSTEM AND DETECTION METHOD**

(57) The present invention provides an active magnetic control capsule robot detection system and detection method, including a first supporting frame, a driving magnet guiding module, a driving magnet module, a detection module, a capsule robot, a second supporting frame and a console. The first supporting frame is configured to install the driving magnet guiding module; a tail end of the driving magnet guiding module is connected with the driving magnet module and the driving magnet guiding module detects position and posture information of the driving magnet module according to information of an encoder in the driving magnet guiding module; the driving magnet module performs magnetic guiding on the capsule robot by generating a magnetic field; the detection module keeps relatively fixed to the driving magnet module and configured to obtain position and posture information of the capsule robot; the second supporting frame is configured to carry a checked body; and the console controls the driving magnet guiding module through a control instruction to drive the driving magnet module to move according to the position and posture information of the driving magnet module and the position and posture information of the capsule robot. By means of the present invention, not only can real-time performance of the system be improved, but also check precision can be guaranteed.

Fig. 1

**Description**

**TECHNICAL FIELD**

The present invention belongs to the technical field of robot position and posture detection, and particularly relates to an active magnetic control capsule robot detection system and detection method.

**BACKGROUND**

In order to realize accurate control over a capsule endoscope, a position and a posture of the capsule endoscope in a human gastrointestinal tract needs to be determined, however, how to realize real-time detection for a magnetic-guiding active capsule endoscope under a high-intensity magnetic field has been a difficulty all the time. A patent whose publication number is CN108354578A and entitled "Capsule Endoscope Positioning System" provides a method by comparing collected digestive tract pictures with data in a network model to realize positioning of the capsule endoscope, however, a structure of an intestinal tract is different from that of a stomach, there are many similar folds and structures in an intestinal tract path, so there are many similarities in the collected pictures, and an accurate result is difficult to obtain by judging the position and the posture of the capsule endoscope in the digestive tract only through data comparison with the network model.

A patent whose publication number is CN103908216A and entitled "Capsule Endoscope System with Magnetic Field Positioning Function and Capsule Endoscope thereof" puts it forward that a magnetic field measurement module is put in the capsule endoscope and the position of the capsule endoscope is judged by measuring a magnetic field intensity of a magnet in vitro and comparing with theoretical magnetic field model data, however, measurement errors of a single magnetic field sensor module under a high-intensity magnetism environment are large, a sensor is prone to measurement failure and saturation, and there is no successfully applied product in the market at present; and moreover, as for a patient, the capsule endoscope is a disposable product, putting modules such as the magnetic field sensor in the capsule endoscope will doubtlessly increase cost of the capsule endoscope and increase economic burden of the patient.

**SUMMARY**

As for the above problems, the present invention provides an active magnetic control capsule robot detection system and detection method so that not only can real-time performance of the system be improved, but also check precision can be guaranteed.

The present invention provides an active magnetic control capsule robot detection system, including a first supporting frame, a driving magnet guiding module, a driving magnet module, a detection module, a capsule robot, a second supporting frame and a console.

The first supporting frame is configured to install the driving magnet guiding module; a tail end of the driving magnet guiding module is connected with the driving magnet module and the driving magnet guiding module detects position and posture information of the driving magnet module; the driving magnet module performs magnetic guiding on the capsule robot by generating a magnetic field, and the capsule robot is provided with a magnet, configured to realize movement control; the detection module keeps relatively fixed to the driving magnet module and configured to obtain position and posture information of the capsule robot; the second supporting frame is configured to carry a checked body in an activity region of the capsule robot; and the console controls the driving magnet guiding module through a control instruction to drive the driving magnet module to move according to the position and posture information of the driving magnet module and the position and posture information of the capsule robot.

On the basis of the above technical solution, the present invention may also make the following improvement.

Furthermore, the detection module of the present invention is mainly composed of magnetic sensors, an installing component, a data processing unit and an inertia detection unit;

the installing component is fixed to the driving magnet module and keeps relatively fixed to the driving magnet module; the magnetic sensors, the data processing unit and the inertia detection unit are fixed to the installing component; and the magnetic sensors are configured to realize measurement of a magnetic field signal, the data processing unit is configured to process a signal measured by the magnetic sensors so as to obtain the position and posture information of the capsule robot, and the inertia detection unit is configured to obtain a movement state of the detection module.

Furthermore, the installing component is of a hollow polyhedral structure and fixed to the outside of the driving magnet module so that a plurality of its surfaces can use a center of the driving magnet module as a center, the magnetic sensors are distributed on the surfaces of the installing component, and by designing distances between the polyhedral surfaces and the center of the driving magnet module, the magnetic sensors can measure a magnetic field signal on each measurement axis within a measuring range of the measurement axis.

Furthermore, the magnetic sensors of the present invention are installed on the installing component and are distributed in a radial pattern with the center of the driving magnet module as their center, and by adjusting a posture of the magnetic sensors relative to a magnetic field direction of their own installing points, it is guaranteed that the magnetic sensors can measure the magnetic field signal within the measuring range in at least one measurement direction.

Furthermore, the magnetic sensors of the present invention are composed of at least one of a uniaxial sensor, a biaxial sensor or a triaxial sensor.

Furthermore, the first supporting frame of the present invention is of an independent structure and provided with rollers and supporting feet at its bottom.

Furthermore, a groove structure configured to contain the driving magnet module is formed in a side surface of the first supporting frame of the present invention, or a containing module is disposed on the first supporting frame and configured to contain the driving magnet module.

Furthermore, a limiting module is installed on the first supporting frame of the present invention and provides a limiting effect on the driving magnet guiding module being in a movement process.

Furthermore, the second supporting frame of the present invention has at least one of a freedom degree of horizontal sliding, a freedom degree of horizontal rotation or a freedom degree of vertical ascending and descending.

Furthermore, the driving magnet guiding module of the present invention is provided with an encoder unit, detecting movement displacement at each freedom degree and configured to detect position and posture information of the driving magnet module.

Furthermore, the driving magnet guiding module has a function of sensing contact force information, and when the driving magnet guiding module senses contact with a checked subject in a using process, the driving magnet guiding module stops moving or returns.

The present invention provides an active magnetic control capsule robot detection method, and its specific process is:

step 1, reading a magnetic field measurement value collected by each magnetic sensor and position and posture data of a detection module;

step 2, obtaining an effective magnetic field value of a capsule robot in a current position and posture according to the magnetic field measurement values;

step 3, calculating position and posture information of the capsule robot according to the active magnetic field value; and

step 4, outputting the position and posture information of the capsule robot to a console when a condition that a position and posture of the capsule robot does not change is met.

The present invention provides an active magnetic control capsule robot detection method, and its specific process is:

step 1, reading a magnetic field measurement value collected by each magnetic sensor and position and posture data of a detection module;

entering step 2 when a condition that a position and posture of a capsule robot does not change is met;

step 2, obtaining an effective magnetic field value of the capsule robot in a current position and posture according to the magnetic field measurement values;

step 3, calculating position and posture information of the capsule robot according to the active magnetic field value; and

step 4, outputting the position and posture information of the capsule robot to a console.

Furthermore, a method of judging the condition that the position and posture of the capsule robot does not change in the present invention is:

when an accelerated velocity deviation of the detection module recorded at different moments is smaller than a set threshold value, it is believed that the position and posture of the capsule robot doe not change in this period of time, that is, calculated position and posture data of the capsule robot are used as an effective result to be output to the console.

Furthermore, a method of judging the condition that the position and posture of the capsule robot does not change in the present invention is:

when a maximum uniaxial magnetic field deviation detected by the detection module and recorded at different moments is smaller than a set threshold value, it is believed that the position and posture of the capsule robot doe not change in this period of time, that is, calculated position and posture data of the capsule robot are used as an effective result to be output to the console.

Furthermore, a method of judging the condition that the position and posture of the capsule robot does not change in the present invention is:

when a position and posture deviation of the detection module recorded at different moments is smaller than a set threshold value, it is believed that the position and posture of the capsule robot doe not change in this period of time, that is, calculated position and posture data of the capsule robot are used as an effective result to be output to the console.

Beneficial effects

By means of the active magnetic control capsule robot detection system and detection method provided by the present invention, not only can real-time performance of the system be improved, but also check precision can be guaranteed.

**BRIEF DESCRIPTION OF THE DRAWINGS**

Fig. 1 is a schematic diagram of a capsule robot magnetic control apparatus with a detection module;
Fig. 2 is a schematic diagram of a relatively fixed position of a detection module being installed in rear of a driving magnet module;
Fig. 3 is a schematic diagram of a capsule robot magnetic control apparatus with a detection module;
Fig. 4 is a schematic diagram of relatively position fixing of a detection module and a driving magnetic module;
Fig. 5 is a schematic diagram of a capsule robot magnetic control apparatus with a detection module;
Fig. 6 is a schematic diagram of arrangement of chips of magnetic sensors; and
Fig. 7 is a schematic diagram of a relation between a coordinate system of chips of magnetic sensors and an external magnet magnetic field.

**DETAILED DESCRIPTION**

To make the objectives, technical solutions and advantages of the embodiments of the present invention clearer, a clear and complete description of the technical solutions in embodiments of the present invention will be given below, in combination with the accompanying drawings in the embodiments of the present invention.

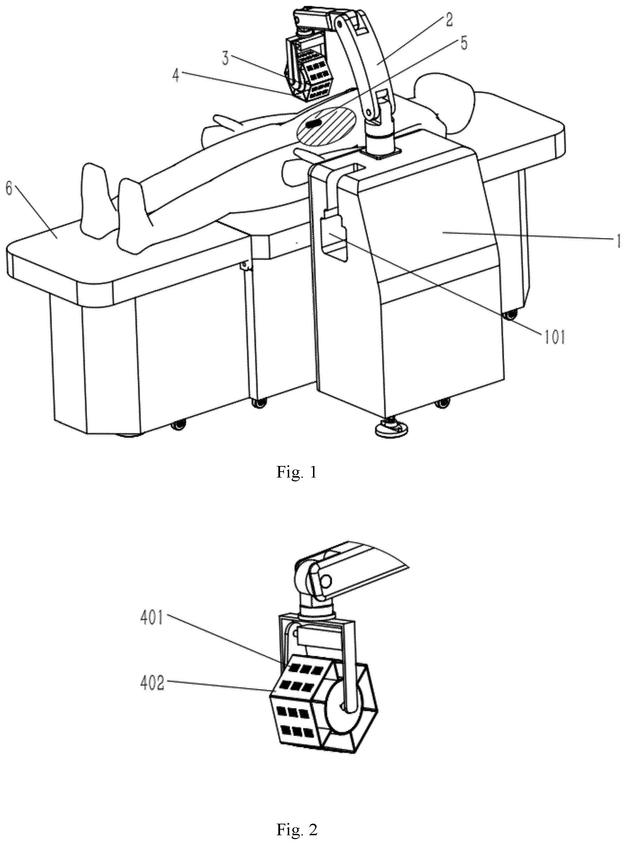

An embodiment of the present invention provides an active magnetic control capsule robot detection system, as shown in Fig. 1, including a first supporting frame 1, a driving magnet guiding module 2, a driving magnet module 3, a detection module 4, a capsule robot 5, a second supporting frame 6 and a console, wherein the console is not shown in Fig. 1.

The first supporting frame 1 is configured to install the driving magnet guiding module 2; a tail end of the driving magnet guiding module 2 is connected with the driving magnet module 3 and detects position and posture information of the driving magnet module according to information of an encoder and the like in the driving magnet guiding module; the console is configured to control the driving magnet guiding module 2 to drive the driving magnet module to perform multi-freedom-degree movement; the driving magnet module 3 performs magnetic guiding on the capsule robot 5 by generating a magnetic field, and the capsule robot 5 contains a tiny magnet and has imaging and data transmission functions; the detection module 4 is fixed to the outside of the driving magnet module 3 and configured to obtain position and posture information of the capsule robot 5; and the second supporting frame 6 is configured to carry a checked body in an activity region of the capsule robot.

In another embodiment of the present invention, on the basis of the above technical solution, the console sends a control instruction to a controller located inside the driving magnet guiding module 2, the controller is driven by the control instruction to drive the driving magnet module 3 to perform multi-freedom-degree movement so as to guide the capsule robot 5 to move along a region of interest.

In another embodiment of the present invention, on the basis of the above technical solution, the driving magnet guiding module 2 is in a form of Cartesian rectangular coordinates, the driving magnet module 3 at the tail end has six freedom degrees, the driving magnet module 3 can be driven by three motors to move linearly along three coordinate axes of X, Y and Z, and the driving magnet module 3 can be driven by three other motors to rotate around the three axes.

As shown in Fig. 1 to Fig. 2, in another embodiment of the present invention, on the basis of the above technical solution, the detection module 4 may be mainly composed of: magnetic sensors 401, an installing component 402, a data processing unit and an inertia detection unit; the installing component 402 is fixed to the driving magnet module 3 and keeps relatively fixed to the driving magnet module 3; the magnetic sensors 401, the data processing unit and the inertia detection unit are fixed to the installing component 402; and the magnetic sensors 401 are configured to realize measurement of a magnetic field signal, the data processing unit is configured to process the signal measured by the magnetic sensors 401 so as to obtain position and posture information of the capsule robot 5, and the inertia detection unit is configured to obtain a movement state of the detection module and may adopt an accelerometer, a gyroscope, etc.

As shown in Fig. 1 to Fig. 2, in another embodiment of the present invention, on the basis of the above technical solution, the magnetic sensors 401 may be composed of: a triaxial magnetic sensor, the installing component 402 is of a hollow polyhedral structure and fixed to the outside of the driving magnetic module 3 so that a plurality of its surfaces can use a center of the driving magnet module 3 as a center, the magnetic sensors 401 are distributed on the surfaces of the installing component 402, and by designing distances between the polyhedral surfaces and the center of the driving magnet module 3, the magnetic sensors 401 can measure a magnetic field signal on each measurement axis within a measuring range of the measurement axis. The data processing unit includes a microprocessor, a data communication first unit, a power supply and a data communication second unit and other units, receives the magnetic field signal obtained by the magnetic sensors and processes the signal so as to obtain the position and posture information of the capsule robot 5.

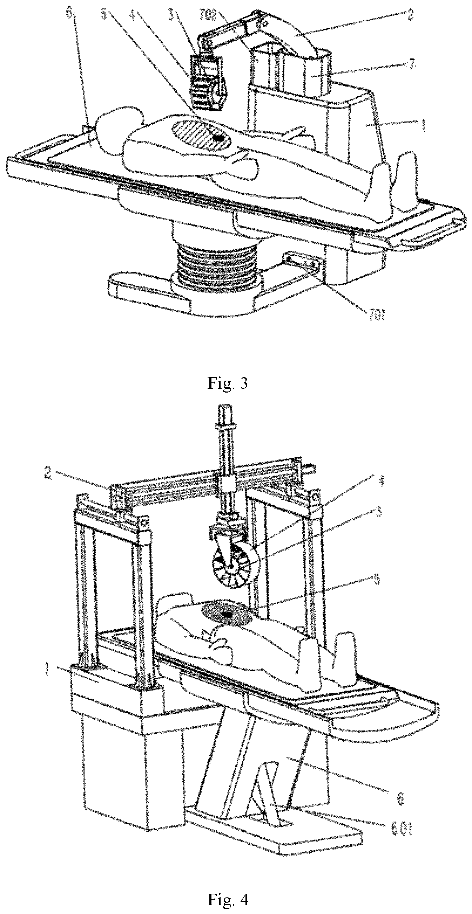

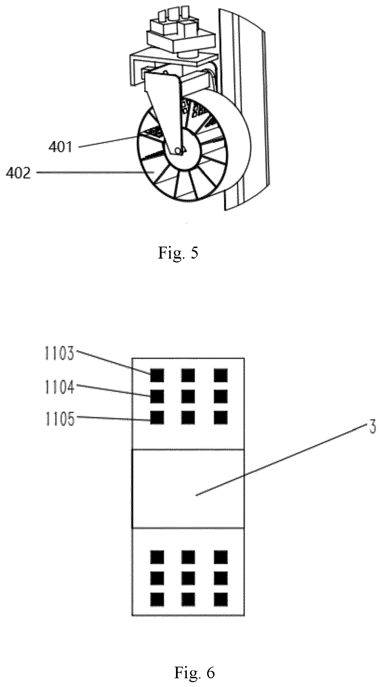

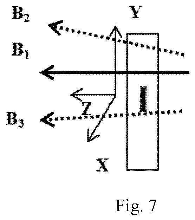

As shown in Fig. 4 to Fig. 6, in another embodiment of the present invention, on the basis of the above technical solution, a layout mode, on the installing component 402, of the magnetic sensors 401 in the detection module 4 differs, the magnetic sensors 401 are installed on a cross-section-type structure of the installing component 402 and use a center of the driving magnet module 3 as their center to be distributed in a radial pattern. By adjusting distances between the magnetic sensors 401 and the center of the driving magnet module 3, part of magnetic sensors 401 can measure the magnetic field signal on each measurement axis within the measuring range of the measurement axis. By adjusting a posture of a certain magnetic sensor 401 relative to a magnetic field direction at an installing point, the sensor can measure the magnetic field signal within a measuring range in at least one measurement direction. As shown in Fig. 6, the three sensors in a row 1103 are far from an external magnet, and three axes do not exceed a sensor measuring range. The magnetic sensors in a row 1104 and row 1105 are close to the external magnet, and by adjusting a direction of the chips of the sensors, the magnetic sensors are distributed in the magnetic field direction, that is, a certain axis of the magnetic sensors approaches to the magnetic field direction, so that the other two axes can measure a magnetic field within the sensor measuring range. As shown in Fig. 7, an axis Z is approximately the same as a magnetic field direction, and the magnetic field is approximately vertical to the X axis and the Y axis.

As shown in Fig. 1, in another embodiment of the present invention, on the basis of the above technical solution, the first supporting frame 1 is of an independent structure and provided with rollers and supporting feet at its bottom, a groove structure 101 may be further formed in a side surface of the first supporting frame 1 and configured to perform isolation treatment on the driving magnet module 3 through a movement instruction or a restoration instruction when a device is idle.

As shown in Fig. 3, in another embodiment of the present invention, on the basis of the above technical solution, a limiting module 7 is installed on the first supporting frame 1 and provides a limiting effect on the driving magnet guiding module 2 in a movement process so that the driving magnet module 3 at the tail end of the driving magnet guiding module and the detection module 4 can be prevented from making contact with the checked body.

As shown in Fig. 3, in another embodiment of the present invention, on the basis of the above technical solution, a containing module 702 is arranged on the first supporting frame 1, may be arranged beside the limiting module 7 of the first supporting frame 1 and is configured to perform isolation containing on the driving magnet module 3 through the movement instruction or the restoration instruction when the device is idle.

As shown in Fig. 4, in another embodiment of the present invention, on the basis of the above technical solution, the first supporting frame 1 may be of a gantry framework structure so as to conveniently place a linear movement sliding rail on a support, a desktop height of the first supporting frame 1 is lower than that of the second supporting frame 6 so that a desktop of the second supporting frame 6 can conveniently extend into a working region.

As shown in Fig. 1, in another embodiment of the present invention, on the basis of the above technical solution, the second supporting frame 6 adopts a split structure capable of being assembled and disassembled, and meanwhile, the feet and the rollers are installed at the bottom of the second supporting frame so that the second supporting frame can conveniently move.

As shown in Fig. 3, in another embodiment of the present invention, on the basis of the above technical solution, the second supporting frame 6 serves as a carrying module of the checked body and has a freedom degree of horizontal sliding, a freedom degree of horizontal rotation and a freedom degree of vertical ascending and descending, wherein the freedom degree of horizontal sliding is realized by making a bed surface move horizontally in a sliding rail direction, the freedom degree of horizontal rotation is realized by making a whole upper bed body rotate around a middle rotating supporting shaft through motor driving, and the freedom degree of ascending and descending movement is realized by a built-in pneumatic cylinder apparatus. Meanwhile, a mechanical joint 701 is arranged between the bottom of the second supporting frame 6 and a bottom of the first supporting frame 1 and configured to realize position fixing between the both.

As shown in Fig. 4, in another embodiment of the present invention, on the basis of the above technical solution, the second supporting frame 6 serves as the carrying module of the checked body and has a freedom degree of horizontal sliding and a freedom degree of ascending and descending movement, wherein the freedom degree of horizontal sliding is realized by making a bed surface move horizontally in a sliding rail direction, and the freedom degree of ascending and descending movement is realized by achieving a supporting effect on an oblique supporting column through a supporting rod 601.

In another embodiment of the present invention, on the basis of the above technical solution, the driving magnet guiding module is provided with an encoder unit, detecting movement displacement at each freedom degree and configured to detect position and posture information of the driving magnet module. Meanwhile, the driving magnet guiding module 2 has a function, on a surface, of sensing contact force information, and can detect contact with a checked subject in a using process, and when the driving magnet guiding module 2 senses contact with the checked subject in the using process, the driving magnet guiding module may stop moving or return.

In another embodiment of the present invention, on the basis of the above technical solution, the console may include: a computer, a mouse, a keyboard, a display, a carrying module and other devices. An operator observes checking images of a gastrointestinal region output from the capsule robot 5 through the display for checking, and according to the position and posture information of the driving magnet module provided by the controller in the driving magnet guiding module 2 and the position and posture information of the capsule robot 5 provided by the detection module 4, a control instruction is generated so that the driving magnet guiding module 2 can drive the driving magnet module 3 to perform multi-freedom-degree movement.

A specific process of an active magnetic control capsule robot detection method of an embodiment of the present invention is: a capsule robot position and posture detection method adopts N triaxial magnetic sensor chips, sensors adopt an arrangement mode shown in Fig. 2, distances between the sensor chips and an external magnet are adjusted, so that all the sensor chips can measure a magnetic field signal on each measurement axis within a measuring range of the measurement axis. If a sensor arrangement mode shown in Fig.5 or Fig. 6 is adopted, only magnetic sensors within the measuring range is adopted to perform magnetic field measurement in an axial direction, other modes are the same as the following embodiment.

Step 1: a magnetic field measurement value collected by each magnetic sensor and position and posture data of a detection module are read.

Detection is started, a moment T1 is recorded, a total magnetic field measured by N magnetic sensor chips starts to be read at the moment T1, recorded as BX(1), BY(1), and BZ(1); BX(2), BY(2) and BZ(2); ..., BX(N), BY(N) and BZ(N) respectively.

Step 2: an effective magnetic field value of a capsule robot in a current position and posture is obtained according to the magnetic field measurement value, specifically: A fixed magnetic field deviation value of the driving magnet module and geomagnetism of the detection module in a current position and posture are subtracted from the magnetic field measurement value collected by each magnetic sensor; and

In any detection process, in a current position and posture of an external magnet and a magnetic sensor array, under a coordinate system of each magnetic sensor chip (different magnetic sensor chips are different magnetic sensor chip coordinate systems), a current environment magnetic field signal is subtracted from a total magnetic field signal obtained from a detection array so as to obtain an effective magnetic field signal, obtained by the detection array, from the capsule robot, the current environment magnetic field signal includes an external driving magnet fixed magnetic field deviation value, a geomagnetic signal of a location where each sensor in the array is located (under the coordinate system of each magnetic sensor chip), etc.

$$BT\_x(i)=BX(i)-BT\_EPMx(i)-BT1\_earthx(i),$$

$$BT\_y(i)=BY(i)-BT\_EPMy(i)-BT1\_earthy(i),$$

and

$$BT\_z(i)=BZ(i)-BT\_EPMz(i)-BT1\_earthz(i),$$

where i=0, 1, 2...N-1, and N is the quantity of triaxial sensors in the array.

BX, BY and BZ here represent a total magnetic field value measured by the triaxial magnetic sensors, BT1_earthx, BT1_earthy and BT_earthz represent a geomagnetic field value in a position and posture at this moment, BT_EPMx, BT_EPMy and BT EPMz represent distribution of fixed external magnet magnetic fields in the array, and BT_x(i), BT_y(i) and BT_z(i) represent effective magnetic field data of each magnetic sensor.

A solving method of a geomagnetic signal under the coordinate systems of the magnetic sensor chips is:

A geomagnetic field signal detected by each sensor is pre-stored uniformly according to a certain density within a space range of array movement under a world coordinate system by using each triaxial magnetic sensor. Under the world coordinate system, each triaxial magnetic sensor is horizontally placed (that is, overlapping according to the world coordinate system and the sensor coordinate system), each magnetic sensor only performs movement in horizontal and vertical directions, each triaxial magnetic sensor is uniformly moved according to a certain density within the space range of array movement, a triaxial geomagnetic signal is uniformly measured and stored, recorded as BT0_earthx(i), BT0_earthy(i), BT0_earthz(i), i=0, 1, 2...M, and M is the quantity of stored magnetic fields. As a magnitude of a geomagnetic signal is in hundreds of mGs, positioning of the capsule robot requires that a magnetic field accuracy of each axis is below dozens of mGs, the stored geomagnetic signal requires that a difference between adjacent geomagnetic signals does not exceed dozens of mGs.

In actual measurement, firstly, a posture of a driving magnet module 3 to the world coordinate system is obtained through conversion by using a posture of a joint coordinate system of a driving magnet guiding module 2 to the world coordinate system at a current moment. As there is a fixed corresponding relation between the array coordinate system and a coordinate system of the driving magnet module 3, a real-time posture of the array coordinate system to the world coordinate system is deduced, and finally, a relation between a coordinate system of each chip and the world coordinate system is deduced. Therefore, geomagnetic signals stored in closest sensor chips or their differences under the world coordinate system are converted into geomagnetic field signals under the magnetic sensor chip coordinate systems. Finally, a geomagnetic field signal of each magnetic sensor under the coordinate system of the magnetic sensor chip at the current moment is obtained.

Step 3: position and posture information of the capsule robot is calculated according to the effective magnetic field value.

Firstly, an effective magnetic field under each magnetic sensor chip coordinate system is converted into an effective magnetic field value under the same array coordinate system by using projection, coordinate system conversion, etc. A position and posture detection algorithm is run through a microprocessor by using the effective magnetic field value under the array coordinate system so as to obtain position and posture data of the capsule robot under the array coordinate system, then the position and posture data are converted into data under the world coordinate system and recorded as current position and posture data of the capsule robot, and this moment is recorded as moment T2.

Step 4, the position and posture data of the capsule robot is output to a console when a condition that a position and posture of the capsule robot does not change is met.

A method of judging the condition that the position and posture of the capsule robot does not change is:
An accelerated velocity a (1) of the detection module starts to be read from the moment T1, in a whole algorithm flow, the accelerated velocity of the detection module is read at intervals of deltaT, it is read and recorded as a(2) at a moment T1+deltaT, and so on, that is, it is read as a(k) at a moment T1+(k-1)*deltaT, it is read as a(k+1) at a moment of T1+k*deltaT, T1+k*deltaT>T2>T1+(k-1)*deltaT, and the accelerated velocity is recorded till a(k+1).

An average value a average of a(1), ...a(k), a(k+1) is calculated, then an absolute value a_s(i) of a deviation is calculated, a maximum value MAX_a in a_s(i) is discovered, when MAX a is smaller than a certain small threshold value, it is believed that the detection module is still in an algorithm operation process, at the moment, data are effective, the calculated position and posture data are output to the console, operation succeeds, and then algorithm operation next time is started. If MAX a is larger than a given threshold value, an operation result at the moment is abandoned, recalculation is performed till MAX a is smaller than the threshold value, operation succeeds, and then algorithm operation next time is started. The threshold value here is obtained according to actual experience.

$$a\_average = (a(1)+a(2)+...a(k+1))/(k+1)$$

and
a_s(i)=|a_average-a(i)|, where i=1, 2, ...k+1.

A specific process of another active magnetic control capsule robot detection method of an embodiment of the present invention is as follows.

Step 1: a magnetic field measurement value collected by each magnetic sensor and position and posture data of a detection module are read.

A magnetic field measured by each of N triaxial magnetic sensor chips is read in sequence, that is, it is read as BX(1), BY(1) and BZ(1) at a moment t1, BX(2), BY(2), BZ(2);..., BX(N), BY(N) and BZ(N).

It enters step 2 when a condition that a position and posture of a capsule robot does not change.

A method of judging the condition that the position and posture of the capsule does not change is:
a magnetic field of N magnetic sensors is measured again, and it is read as BX(1)', BY(1)' and BZ(1)' at a moment t1'; then BX(2)', BY(2)' and BZ(2)'; ...; BX(N)', BY(N)', BZ(N)'. A difference value is calculated, as shown in the following:

BX_ERROR(i)=|BX(i)-BX(i)'|,
BY_ERROR(i)=|BY(i)-BY(i)'|, and
BZ_ERROR(i)=|BZ(i)-BZ(i)'|, where i=1, 2, ...N.

When a maximum value in BX ERROR(i) BY ERROR(i) and BZ ERROR(i) is smaller than a given threshold value, it is believed that a measured magnetic field value this time is effective, a magnetic field value is recorded, and next step is continued. Otherwise, the measured value this time is abandoned, step 1 is repeated till the maximum value in BX-ERROR(i), BY ERROR(i) and BZ ERROR(i) is smaller than the given threshold value, and then the next step is continued. The given threshold value here is about dozens of mGS depending on an actual experiment result precision, an effective magnetic field size, magnetic sensor chips and other factors.

Step 2 and step 3 are the same as those of the active magnetic control capsule robot detection method in the last embodiment.

Step 4, the calculated position and posture data of the capsule robot are output to a console.

To sum up, the above is only preferred embodiments of the present invention rather than intends to limit the protection scope of the present invention. Those skilled in the art can easily figure out other embodiments and modifications thereof, and any modification, equivalent replacement, improvement, etc. within the spirit and principle of the present invention fall within the protection scope of the present invention.

## Claims

1. An active magnetic control capsule robot detection system, **characterized by** comprising a first supporting frame (1), a driving magnet guiding module (2), a driving magnet module (3), a detection module (4), a capsule robot (5), a second supporting frame (6) and a console, wherein

   the first supporting frame (1) is configured to install the driving magnet guiding module (2); a tail end of the driving magnet guiding module (2) is connected with the driving magnet module (3) and the driving magnet guiding module detects position and posture information of the driving magnet module (3); the driving magnet module (3) performs magnetic guiding on the capsule robot (5) by generating a magnetic field, and the capsule robot (5) is provided with a magnet, configured to realize movement control; the detection module (4) keeps relatively fixed to the driving magnet module (3) and configured to obtain position and posture information of the capsule robot (5); the second supporting frame (6) is configured to carry a checked body in an activity region of the capsule robot; and the console controls the driving magnet guiding module (2) through a control instruction to drive the driving magnet module to move according to the position and posture information of the driving magnet module (3) and the position and posture information of the capsule robot (5).

2. The active magnetic control capsule robot detection system of claim 1, **characterized in that** the detection module (4) is mainly composed of magnetic sensors (401), an installing component (402), a data processing unit and an inertia detection unit; the installing component (402) is fixed to the driving magnet module (3) and keeps relatively fixed to the driving magnet module (3); the magnetic sensors (401), the data processing unit and the inertia detection unit are fixed to the installing component (402); and the magnetic sensors (401) are configured to realize measurement of a magnetic field signal, the data processing unit is configured to process a signal measured by the magnetic sensors (401) so as to obtain the position and posture information of the capsule robot (5), and the inertia detection unit is configured to obtain a movement state of the detection module.

3. The active magnetic control capsule robot detection system of claim 1, **characterized in that** an installing component (402) is of a hollow polyhedral structure and fixed to the outside of the driving magnet module (3) so that a plurality of its surfaces can use a center of the driving magnet module (3) as a center, magnetic sensors (401) are distributed on the surfaces of the installing component (402), and by designing distances between the polyhedral surfaces and the center of the driving magnet module (3), the magnetic sensors (401) can measure a magnetic field signal on each measurement axis within a measuring range of the measurement axis.

4. The active magnetic control capsule robot detection system of claim 1, **characterized in that** magnetic sensors (401) are installed on an installing component (402) and are distributed in a radial pattern with the center of the driving magnet module (3) as their center, and by adjusting a posture of the magnetic sensors relative to a magnetic field direction of their own installing points, it is guaranteed that the magnetic sensors can measure the magnetic field signal within the measuring range in at least one measurement direction.

5. The active magnetic control capsule robot detection system of claim 1, **characterized in that** magnetic sensors are composed of at least one of a uniaxial sensor, a biaxial sensor or a triaxial sensor.

6. The active magnetic control capsule robot detection system of claim 1, **characterized in that** a groove structure configured to contain the driving magnet module (3) is formed in a side surface of the first supporting frame (1), or a containing module (702) is disposed on the first supporting frame (1) and configured to contain the driving magnet module (3).

7. The active magnetic control capsule robot detection system of claim 1, **characterized in that** a limiting module (7) is installed on the first supporting frame (1) and provides a limiting effect on the driving magnet guiding module (2) being in a movement process.

8. The active magnetic control capsule robot detection system of claim 1, **characterized in that** the second supporting frame (6) has at least one of a freedom degree of horizontal sliding, a freedom degree of horizontal rotation or a freedom degree of vertical ascending and descending.

9. The active magnetic control capsule robot detection system of claim 1, **characterized in that** the driving magnet guiding module (2) is provided with an encoder unit, detecting movement displacement at each freedom degree and configured to detect position and posture information of the driving magnet module.

10. The active magnetic control capsule robot detection system of claim 1, **characterized in that** the driving magnet guiding module (2) has a function of sensing contact force information, and when the driving magnet guiding module (2) senses contact with a checked subject in a using process, the driving magnet guiding module stops moving or returns.

11. An active magnetic control capsule robot detection method, wherein its specific process is:

    Step 1, reading a magnetic field measurement value collected by each magnetic sensor and position and posture data of a detection module;
    step 2, obtaining an effective magnetic field value of a capsule robot in a current position and posture according to the magnetic field measurement values;
    step 3, calculating position and posture information of the capsule robot according to the active magnetic field value; and
    step 4, outputting the position and posture information of the capsule robot to a console when a condition that a position and posture of the capsule robot does not change is met.

12. An active magnetic control capsule robot detection method, wherein its specific process is:

    step 1, reading a magnetic field measurement value collected by each magnetic sensor and position and posture data of a detection module;
    entering step 2 when a condition that a position and posture of a capsule robot does not change is met;
    step 2, obtaining an effective magnetic field value of the capsule robot in a current position and posture according to the magnetic field measurement values;
    step 3, calculating position and posture information of the capsule robot according to the active magnetic field value; and
    step 4, outputting the position and posture information of the capsule robot to a console.

13. The active magnetic control capsule robot detection method of claim 11 or 12, **characterized in that** a method of judging the condition that the position and posture of the capsule robot does not change is:
when an accelerated velocity deviation of the detection module recorded at different moments is smaller than a set threshold value, it is believed that the position and posture of the capsule robot doe not change in this period of time, that is, calculated position and posture data of the capsule robot are used as an effective result to be output to the console.

14. The active magnetic control capsule robot detection method of claim 11 or 12, **characterized in that** a method of judging the condition that the position and posture of the capsule robot does not change is:
when a maximum uniaxial magnetic field deviation detected by the detection module and recorded at different moments is smaller than a set threshold value, it is believed that the position and posture of the capsule robot doe not change in this period of time, that is, calculated position and posture data of the capsule robot are used as an effective result to be output to the console.

15. The active magnetic control capsule robot detection method of claim 11 or 12, **characterized in that** a method of judging the condition that the position and posture of the capsule robot does not change is:

when a position and posture deviation of the detection module recorded at different moments is smaller than a set threshold value, it is believed that the position and posture of the capsule robot doe not change in this period of time, that is, calculated position and posture data of the capsule robot are used as an effective result to be output to the console.

Fig. 1

Fig. 2

Fig. 3

Fig. 4

401

402

Fig. 5

1103

1104

1105

3

Fig. 6

Fig. 7

## INTERNATIONAL SEARCH REPORT

| International application No. |
| --- |
| **PCT/CN2020/094410** |

**A. CLASSIFICATION OF SUBJECT MATTER**

A61B 1/04(2006.01)i

According to International Patent Classification (IPC) or to both national classification and IPC

**B. FIELDS SEARCHED**

Minimum documentation searched (classification system followed by classification symbols)

A61B 1/-

Documentation searched other than minimum documentation to the extent that such documents are included in the fields searched

Electronic data base consulted during the international search (name of data base and, where practicable, search terms used)

CNPAT, CNKI, WPI, EPODOC: 北京理工大学, 磁, 内窥镜, 内镜, 胶囊, 位置, 姿势, 位姿, 控制, 磁传感器, magnet, endoscope, capsule, posture, position, pose, control, sensor

**C. DOCUMENTS CONSIDERED TO BE RELEVANT**

| Category* | Citation of document, with indication, where appropriate, of the relevant passages | Relevant to claim No. |
| --- | --- | --- |
| PX | CN 110236474 A (BEIJING INSTITUTE OF TECHNOLOGY) 17 September 2019 (2019-09-17)<br>  claims 1-15 | 1-15 |
| Y | CN 103932654 A (SHANGHAI JIAO TONG UNIVERSITY) 23 July 2014 (2014-07-23)<br>  description, paragraphs [0021]-[0042], and figures 1-3 | 1-15 |
| Y | CN 108827133 A (BEIJING INSTITUTE OF TECHNOLOGY) 16 November 2018 (2018-11-16)<br>  description, paragraphs [0024]-[0040], and figures 1-3 | 1-15 |
| A | CN 103169443 A (HARBIN INSTITUTE OF TECHNOLOGY SHENZHEN GRADUATE SCHOOL) 26 June 2013 (2013-06-26)<br>  entire document | 1-15 |
| A | JP 2009045279 A (HOYA CORPORATION) 05 March 2009 (2009-03-05)<br>  entire document | 1-15 |
| A | CN 108451490 A (CHONGQING JINSHAN MEDICAL INSTRUMENT CO., LTD.) 28 August 2018 (2018-08-28)<br>  entire document | 1-15 |

☑ Further documents are listed in the continuation of Box C.   ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 August 2020** | **01 September 2020** |

| Name and mailing address of the ISA/CN | Authorized officer |
| --- | --- |
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/094410**

**C.    DOCUMENTS CONSIDERED TO BE RELEVANT**

| Category* | Citation of document, with indication, where appropriate, of the relevant passages | Relevant to claim No. |
|-----------|-----------------------------------------------------------------------------------|-----------------------|
| A | WO 2016067802 A1 (OLYMPUS CORPORATION) 06 May 2016 (2016-05-06)<br>    entire document | 1-15 |
| A | CN 105852783 A (CHONGQING JINSHAN SCIENCE & TECHNOLOGY (GROUP) CO., LTD.) 17 August 2016 (2016-08-17)<br>    entire document | 1-15 |

Form PCT/ISA/210 (second sheet) (January 2015)

**INTERNATIONAL SEARCH REPORT**
Information on patent family members

International application No.

**PCT/CN2020/094410**

| Patent document cited in search report | | | Publication date (day/month/year) | Patent family member(s) | | | Publication date (day/month/year) |
|---|---|---|---|---|---|---|---|
| CN | 110236474 | A | 17 September 2019 | None | | | |
| CN | 103932654 | A | 23 July 2014 | CN | 103932654 | B | 04 November 2015 |
| CN | 108827133 | A | 16 November 2018 | CN | 108827133 | B | 01 May 2020 |
| CN | 103169443 | A | 26 June 2013 | None | | | |
| JP | 2009045279 | A | 05 March 2009 | None | | | |
| CN | 108451490 | A | 28 August 2018 | None | | | |
| WO | 2016067802 | A1 | 06 May 2016 | JP | 6022112 | B2 | 09 November 2016 |
| | | | | JP | WO2016067802 | A1 | 27 April 2017 |
| CN | 105852783 | A | 17 August 2016 | CN | 105852783 | B | 30 October 2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108354578 A
- CN 103908216 A